(19)

**Europäisches Patentamt**
**European Patent Office**
**Office européen des brevets**

(11) **EP 4 522 993 B1**

(12) **EUROPEAN PATENT SPECIFICATION**

(45) Date of publication and mention
of the grant of the patent:
**15.04.2026  Bulletin 2026/16**

(21) Application number: **23804411.9**

(22) Date of filing: **26.04.2023**

(51) International Patent Classification (IPC):
*G01N 33/58* (2006.01)     *G01N 33/573* (2006.01)
*C12N 15/62* (2006.01)     *C12N 15/63* (2006.01)
*G06V 20/69* (2022.01)     *G06T 7/00* (2017.01)
*G06V 10/143* (2022.01)     *G06V 10/774* (2022.01)

(52) Cooperative Patent Classification (CPC):
**G01N 33/582; G06T 7/0014; G06V 10/143;**
**G06V 10/774; G06V 20/693; G06V 20/695;**
**G06V 20/698;** G06T 2207/10056;
G06T 2207/10064; G06T 2207/20081;
G06T 2207/20084; G06T 2207/30024

(86) International application number:
**PCT/US2023/066234**

(87) International publication number:
**WO 2023/220522 (16.11.2023 Gazette 2023/46)**

(54) **METHOD FOR MONITORING LIVE CELLS**

VERFAHREN ZUR ÜBERWACHUNG LEBENDER ZELLEN

PROCÉDÉ DE SURVEILLANCE DE CELLULES VIVANTES

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **09.05.2022  US 202217740313**

(43) Date of publication of application:
**19.03.2025  Bulletin 2025/12**

(60) Divisional application:
**25195081.2 / 4 636 091**

(73) Proprietor: **Sartorius BioAnalytical Instruments,**
**Inc.**
**Bohemia, NY 11716 (US)**

(72) Inventors:
• **SÖRMAN PAULSSON, Elsa**
**903 33 Umea (SE)**
• **EDLUND, Christoffer**
**903 33 Umea (SE)**
• **FILONOV, Grigory**
**Ann Arbor, MI 48103 (US)**

• **SCHRAMM, Cicely**
**Ann Arbor, MI 48103 (US)**
• **SJÖGREN, Rickard**
**903 33 Umea (SE)**

(74) Representative: **Novagraaf International SA**
**Chemin de l'Echo 3**
**1213 Onex, Geneva (CH)**

(56) References cited:
**US-A1- 2021 325 309**

• **DAVID A. VAN VALEN ET AL: "Deep Learning**
**Automates the Quantitative Analysis of**
**Individual Cells in Live-Cell Imaging**
**Experiments", PLOS COMPUTATIONAL**
**BIOLOGY, vol. 12, no. 11, 4 November 2016**
**(2016-11-04), pages e1005177, XP055503466,**
**DOI: 10.1371/journal.pcbi.1005177**

EP 4 522 993 B1

- HELGADOTTIR SAGA ET AL: "Extracting quantitative biological information from bright-field cell images using deep learning", vol. 2, no. 3, 20 July 2021 (2021-07-20), XP093309184, ISSN: 2688-4089, Retrieved from the Internet <URL:https://pmc.ncbi.nlm.nih.gov/articles/PMC10903417/pdf/BRIEIM-000002-031401_1.pdf> [retrieved on 20250828], DOI: 10.1063/5.0044782
- ALI MOHAMMED A.S. ET AL: "Evaluating Very Deep Convolutional Neural Networks for Nucleus Segmentation from Brightfield Cell Microscopy Images", vol. 26, no. 9, 1 October 2021 (2021-10-01), pages 1125 - 1137, XP093309199, ISSN: 2472-5552, Retrieved from the Internet <URL:https://www.sciencedirect.com/science/article/pii/S2472555222067557> [retrieved on 20250828], DOI: 10.1177/24725552211023214
- DAVID A. VAN VALEN, TAKAMASA KUDO, KEARA M. LANE, DEREK N. MACKLIN, NICOLAS T. QUACH, MIALY M. DEFELICE, INBAL MAAYAN, YU TANOUCHI: "Deep Learning Automates the Quantitative Analysis of Individual Cells in Live-Cell Imaging Experiments", PLOS COMPUTATIONAL BIOLOGY, vol. 12, no. 11, pages e1005177, XP055503466, DOI: 10.1371/journal.pcbi.1005177
- LOGG KATARINA, BODVARD KRISTOFER, BLOMBERG ANDERS, KÃ¤LL MIKAEL: "Investigations on light-induced stress in fluorescence microscopy using nuclear localization of the transcription factor Msn2p as a reporter", FEMS YEAST RESEARCH, WILEY-BLACKWELL PUBLISHING LTD., GB, NL, vol. 9, no. 6, 1 September 2009 (2009-09-01), GB, NL , pages 875 - 884, XP093113058, ISSN: 1567-1356, DOI: 10.1111/j.1567-1364.2009.00536.x

**Description**

The present application is an international application claiming priority to U.S. application no. 17/740,313, filed May 9, 2022.

**BACKGROUND**

Many signaling pathways promote cell survival and growth, and their dysregulation is associated with, for example, cancer initiation, progression, and recurrence. Standard methods of evaluating signaling pathways in cells are end point assays which require cell lysis and often involve time-consuming sample preparation and/or assay workflows. DAVID A. VAN VALEN ET AL: "Deep Learning Automates the Quantitative Analysis of Individual Cells in Live-Cell Imaging Experiments", PLOS COMPUTATIONAL BIOLOGY, vol. 12, no. 11, 4 November 2016 (2016-11-04), DOI: 10.1371/journal.pcbi.1005177 is acknowledged as prior art.

**SUMMARY**

A first example includes a method for monitoring one or more live cells, the method comprising: (a) capturing a non-fluorescence image of a sample that includes one or more live cells, wherein the one or more live cells contain fluorescent protein-based nuclear translocation reporters; (b) capturing a fluorescence image of the fluorescent protein-based nuclear translocation reporters in the one or more live cells in the sample; (c) identifying, via a computational model, nuclear pixels of the non-fluorescence image that correspond to nuclei of the one or more live cells; (d) identifying, based on the nuclear pixels, first pixels of the fluorescence image that correspond to the nuclei and second pixels of the fluorescence image that do not correspond to the nuclei; and (e) calculating, based on first intensities of the first pixels and second intensities of the second pixels, a metric representing a first amount of the fluorescent protein-based nuclear translocation reporters located within the nuclei of the one or more live cells and a second amount of the fluorescent protein-based nuclear translocation reporters not located within the nuclei of the one or more live cells.

A second example includes a non-transitory computer readable medium storing instructions that, when executed by a computing device, cause the computing device to perform functions comprising: (a) capturing a non-fluorescence image of a sample that includes one or more live cells, wherein the one or more live cells contain fluorescent protein-based nuclear translocation reporters; (b) capturing a fluorescence image of the fluorescent protein-based nuclear translocation reporters in the one or more live cells in the sample; (c) identifying, via a computational model, nuclear pixels of the non-fluorescence image that correspond to nuclei of the one or more live cells; (d) identifying, based on the nuclear pixels, first pixels of the fluorescence image that correspond to the nuclei and second pixels of the fluorescence image that do not correspond to the nuclei; and (e) calculating, based on first intensities of the first pixels and second intensities of the second pixels, a metric representing a first amount of the fluorescent protein-based nuclear translocation reporters located within the nuclei of the one or more live cells and a second amount of the fluorescent protein-based nuclear translocation reporters not located within the nuclei of the one or more live cells.

A third example includes a system comprising: an optical microscope; a fluorescence microscope; one or more processors; and a non-transitory computer readable medium storing instructions that, when executed by the one or more processors, cause the system to perform functions comprising: (a) capturing a non-fluorescence image of a sample that includes one or more live cells, wherein the one or more live cells contain fluorescent protein-based nuclear translocation reporters; (b) capturing a fluorescence image of the fluorescent protein-based nuclear translocation reporters in the one or more live cells in the sample; (c) identifying, via a computational model, nuclear pixels of the non-fluorescence image that correspond to nuclei of the one or more live cells; (d) identifying, based on the nuclear pixels, first pixels of the fluorescence image that correspond to the nuclei and second pixels of the fluorescence image that do not correspond to the nuclei; and (e) calculating, based on first intensities of the first pixels and second intensities of the second pixels, a metric representing a first amount of the fluorescent protein-based nuclear translocation reporters located within the nuclei of the one or more live cells and a second amount of the fluorescent protein-based nuclear translocation reporters not located within the nuclei of the one or more live cells.

A fourth example includes a method for training a computational model, the method comprising: generating first labels for first pixels of fluorescence images of samples, wherein the first labels indicate whether the first pixels represent a nucleus within the samples; generating, based on the first labels, second labels for second pixels of first non-fluorescence images of the samples, wherein the second labels indicate whether the second pixels represent a nucleus within the samples; and training a computational model to identify pixels of second non-fluorescence images that represent nuclei using the second labels and the first non-fluorescence images.

A fifth example includes a non-transitory computer readable medium storing instructions that, when executed by a computing device, cause the computing device to perform functions comprising: generating first labels for first pixels of fluorescence images of samples, wherein the first labels indicate whether the first pixels represent a nucleus within the samples; generating, based on the first labels, second labels for second pixels of first non-fluorescence images of the samples, wherein the second labels indicate whether the second pixels represent a nucleus within the samples; and training a computational model to identify pixels of second non-fluorescence images that represent nuclei using the second labels and the first non-fluorescence images.

A sixth example includes a system comprising: one or more processors; and a non-transitory computer readable medium storing instructions that, when executed by the one or more processors, cause the system to perform functions comprising: generating first labels for first pixels of fluorescence images of samples, wherein the first labels indicate whether the first pixels represent a nucleus within the samples; generating, based on the first labels, second labels for second pixels of first non-fluorescence images of the samples, wherein the second labels indicate whether the second pixels represent a nucleus within the samples; and training a computational model to identify pixels of second non-fluorescence images that represent nuclei using the second labels and the first non-fluorescence images.

When the term "substantially," "approximately," or "about" is used herein, it is meant that the recited characteristic, parameter, or value need not be achieved exactly, but that deviations or variations, including, for example, tolerances, measurement error, measurement accuracy limitations, and other factors known to those of skill in the art may occur in amounts that do not preclude the effect the characteristic was intended to provide. In some examples disclosed herein, "substantially," "approximately," or "about" means within +/- 0-5% of the recited value.

These, as well as other aspects, advantages, and alternatives will become apparent to those of ordinary skill in the art by reading the following detailed description, with reference where appropriate to the accompanying drawings. Further, it should be understood that this summary and other descriptions and figures provided herein are intended to illustrate by way of example only and, as such, that numerous variations are possible.

## BRIEF DESCRIPTION OF THE DRAWINGS

Figure 1 is a block diagram of an operating environment, according to an example.
Figure 2 is a block diagram of a computing device, according to an example.
Figure 3 is a fluorescence image, according to an example.
Figure 4 is a binary map, according to an example.
Figure 5 is a binary map, according to an example.
Figure 6 is a non-fluorescence image, according to an example.
Figure 7 is a block diagram of a method for training a computational model, according to an example.
Figure 8 is a schematic representation of a non-fluorescence image, according to an example.
Figure 9 is a schematic representation of a fluorescence image, according to an example.
Figure 10 is a block diagram of a method for monitoring a cell, according to an example.
Figure 11 shows phase images, expected nuclei maps, and predicted nuclei maps, according to an example.
Figure 12 is a scatter plot of predicted nuclei area vs. area of target nuclei, according to an example.
Figure 13 is a scatter plot of predicted nuclei area vs. area of target nuclei, according to an example.
Figure 14 is a scatter plot of ratio for predicted nuclei vs. ratio for target nuclei, according to an example.
Figure 15 is a scatter plot of ratio for predicted nuclei vs. ratio for target nuclei, according to an example.
Figure 16 is a scatter plot of ratio for predicted nuclei vs. ratio for target nuclei, according to an example.
Figure 17 is a scatter plot of ratio for predicted nuclei vs. ratio for target nuclei, according to an example.

## DETAILED DESCRIPTION

As discussed above, improved techniques for evaluating signaling pathways within live cells are needed. This disclosure includes a method for monitoring one or more live cells. The method includes capturing a non-fluorescence image (e.g., a bright field image, a dark field image, or a phase contrast image) of a sample that includes the one or more live cells. The one or more live cells contain fluorescent protein-based nuclear translocation reporters (FTRs) (e.g., cell membranes or cell walls of the one or more live cells surround FTRs). The fluorescent protein-based nuclear translocation reporters may be any such reporter that shuttles into and/or out of the nucleus in response to a stimulus of interest, as described in more detail below. The method also includes capturing a fluorescence image of the fluorescent protein-based nuclear translocation reporters in the one or more live cells in the sample. This generally involves illuminating the one or more live cells and selectively detecting the fluorescence emitted by the protein-based nuclear translocation reporters. In one embodiment, the fluorescence image is captured using the same field of view as the non-fluorescence image. The method also includes identifying, via a computational model, nuclear pixels of the non-fluorescence image that correspond to nuclei of the one or more live cells. Any suitable computational model may be used, including but not limited to a vision transformer (ViT), a convolutional neural network or another artificial neural network. The method also includes identifying, based on the nuclear pixels, first pixels of the fluorescence image that correspond to the nuclei and second pixels of the fluorescence image that do not correspond to the nuclei. The method also includes calculating, based on first intensities of the first pixels and second intensities of the second pixels, a metric representing a first amount of the fluorescent protein-based nuclear translocation reporters located within the nuclei of the one or more live cells and a second amount of the fluorescent protein-based nuclear translocation reporters not located within the nuclei of the one or more live cells. Thus, areas of the fluorescence image pertaining to nuclei can be identified without using a separate fluorescent marker to label the nucleus. This greatly simplifies the image analysis, and frees up a fluorescent channel for analysis of another aspect of the cells as deemed appropriate for an intended use (e.g., tracking the locations and dynamics of proteins, organelles, and other cellular components).

The computational model is generally trained to recognize nuclei within non-fluorescence images before being used to classify pixels of unlabeled non-fluorescence images. Thus, a method for training the computational model incudes generating first labels for first pixels of fluorescence images of samples, where the first labels indicate whether the first pixels represent a nucleus within the samples. The first labels can take the form of a binary map and/or can be generated via a thresholding process, for example. The method also includes generating, based on the first labels, second labels for second pixels of first non-fluorescence images of the samples, where the second labels indicate whether the second pixels represent a nucleus within the samples. In one embodiment, this can be done by applying the binary map to the first non-fluorescence images. The method also includes training the computational model to identify pixels of second non-fluorescence images that represent nuclei using the second labels and the first non-fluorescence images

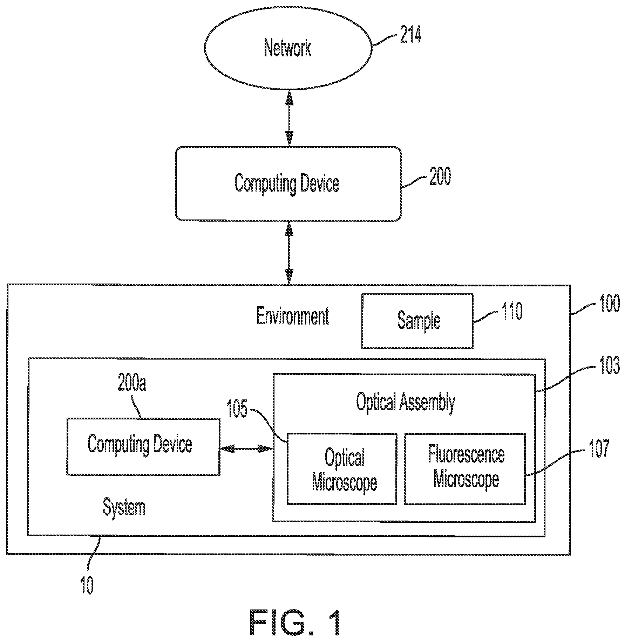

Figure 1 is a block diagram showing an exemplary operating environment 100 of the disclosure that includes a system 10 and a sample 110. The system 10 includes a computing device 200a and an optical assembly 103 that includes an optical microscope 105 and a fluorescence microscope 107. Also shown is a computing device 200 and a network 214, which is described in more detail below.

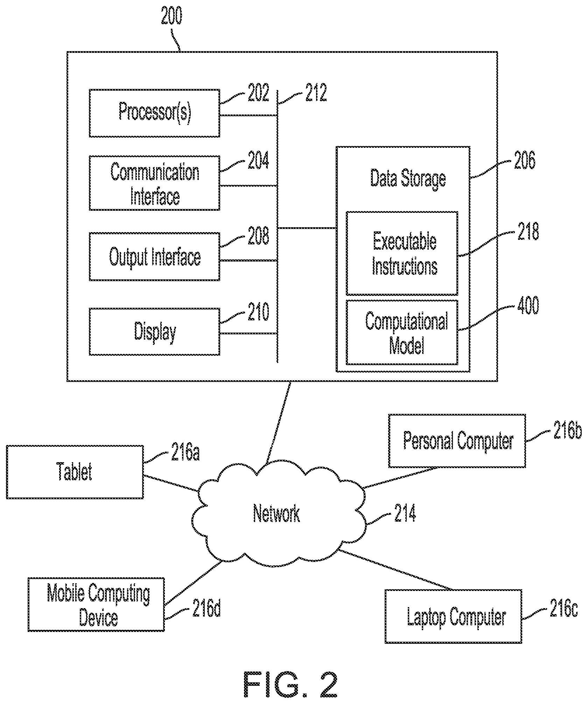

Figure 2 is a block diagram illustrating an exemplary computing device 200 that is configured to interface with operating environment 100, either directly or indirectly. In particular, the computing device 200 can be configured to perform one or more, functions, including image generating functions that are based, in part, on images obtained by the optical microscope 105 and/or the fluorescence microscope 107. The computing device 200 has a processor(s) 202, and also a communication interface 204, data storage 206, an output interface 208, and a display 210 each connected to a communication bus 212. The computing device 200 may also include hardware to enable communication within the computing device 200 and between the computing device 200 and other devices (e.g. not shown). The hardware may include transmitters, receivers, and antennas, for example.

The communication interface 204 may be a wireless interface and/or one or more wired interfaces that allow for both short-range communication and long-range communication to one or more networks 214 or to one or more remote computing devices 216 (e.g., a tablet 216a, a personal computer 216b, a laptop computer 216c and a mobile computing device 216d, for example). Such wireless interfaces may provide for communication under one or more wireless communication protocols, such as Bluetooth, WiFi (e.g., an institute of electrical and electronic engineers (IEEE) 802.11 protocol), Long-Term Evolution (LTE), cellular communications, near-field communication (NFC), and/or other wireless communication protocols. Such wired interfaces may include Ethernet interface, a Universal Serial Bus (USB) interface, or similar interface to communicate via a wire, a twisted pair of wires, a coaxial cable, an optical link, a fiber-optic link, or other physical connection to a wired network. Thus, the communication interface 204 may be configured to receive input data from one or more devices and may also be configured to send output data to other devices.

The communication interface 204 may also include a user-input device, such as a keyboard, a keypad, a touch screen, a touch pad, a computer mouse, a track ball and/or other similar devices, for example.

The data storage 206 may include or take the form of one or more computer-readable storage media that can be read or accessed by the processor(s) 202. The computer-readable storage media can include volatile and/or non-volatile storage components, such as optical, magnetic, organic or other memory or disc storage, which can be integrated in whole or in part with the processor(s) 202. The data storage 206 is considered non-transitory computer readable media. In some examples, the data storage 206 can be implemented using a single physical device (e.g., one optical, magnetic, organic or other memory or disc storage unit), while in other examples, the data storage 206 can be implemented using two or more physical devices.

The data storage 206 thus includes executable instructions 218 stored thereon. The instructions 218 include computer executable code. When the instructions 218 are executed by the processor(s) 202, the processor(s) 202 are caused to perform functions such as any of the functionality described herein. The data storage 206 also includes a computational model 400 stored thereon.

The processor(s) 202 may be a general-purpose processor or a special purpose processor (e.g., digital signal processors, application specific integrated circuits, etc.). The processor(s) 202 may receive inputs from the communication interface 204 and process the inputs to generate outputs that are stored in the data storage 206 and output to the display 210. The processor(s) 202 can be configured to execute the executable instructions 218 (e.g., computer-readable program instructions) that are stored in the data storage 206 and are executable to provide the functionality of the computing device 200 described herein.

The output interface 208 provides information to the display 210 or to other components as well. Thus, the output interface 208 may be similar to the communication interface 204 and can be a wireless interface (e.g., transmitter) or a wired interface as well. The output interface 208 may send commands to one or more controllable devices, for example

The computing device 200 shown in Figure 2 may also be representative of a local computing device 200a in operating environment 100, for example, in communication with the optical microscope 105 and/or fluorescence microscope 107. This local computing device 200a may perform one or more of the steps of the methods described below, may receive input from a user and/or may send image data and user input to computing device 200 to perform all or some of the steps of methods. In addition, in one optional example embodiment, the Incucyte® platform may be utilized to perform methods and includes the combined functionality of computing device 200, the optical microscope 105, and the fluorescence microscope 107.

**Computational Model Training**

The methods of the disclosure comprise identifying, via a computational model, nuclear pixels of the non-fluorescence image that correspond to nuclei of the one or more live cells. The computational model is one that has been trained to identify nuclei without using a nuclear label.

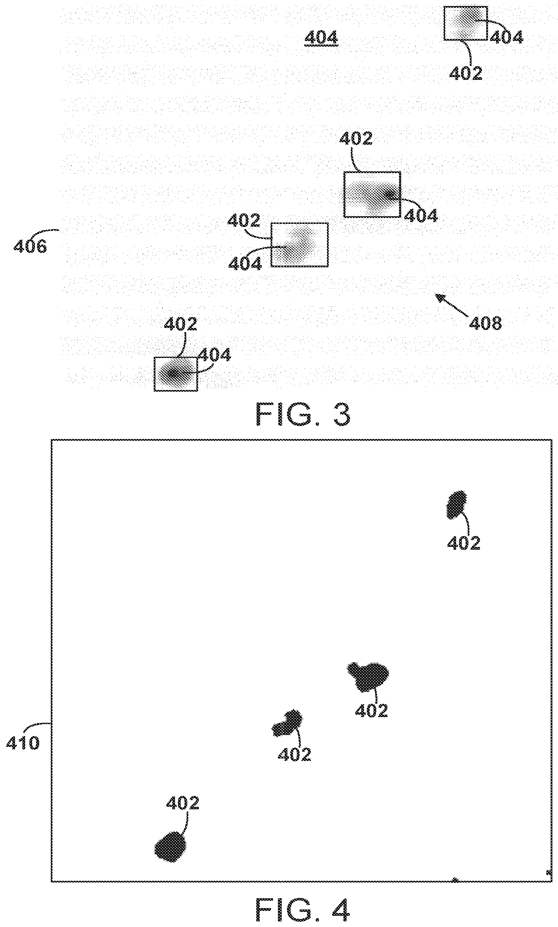

Figure 3 shows an exemplary functionality related to using fluorescence images to train the computational model 400 to identify pixels of non-fluorescence images that correspond to nuclei. The computational model 400 can be stored on the data storage 206 of the computing device 200, for example.

The computing device 200 can generate first labels 402 for first pixels 404 of fluorescence images 406 of samples 408. The first labels 402 indicate whether the first pixels 404 represent a nucleus within the samples 408. The fluorescence images 406 are generally captured subsequent to or contemporaneously with illuminating the samples 408, which causes fluorescent nuclear markers within the samples 408 to emit light, thereby indicating the position of nuclei within the samples 408. While capturing the fluorescence images 406, light that does not correspond to the emission wavelength range of the fluorescent markers is generally filtered out so that only light fluoresced by the fluorescent markers (e.g., the nuclei) is depicted in the fluorescence images 406.

Figure 3 shows a fluorescence image 406 that shows one sample 408 that includes several (e.g., living) cells and corresponding nuclei depicted by the first pixels 404 enclosed by the first labels 402. The fluorescence image 406 in Figure 3 serves as an example for many fluorescence images 406 of many samples 408. The fluorescence image 406 also includes some first pixels 404 that are positioned outside of the first labels 402. In this example, a user manually reviews many fluorescence images 406 and manually generates the first labels 402 with a user interface (for example, using a click and drag motion to draw rectangles around some of the first pixels 404). In this way, the first labels 402 can be created in the form of metadata that indicates pixel locations that correspond to nuclei of the cells. It can also generally be inferred that the first pixels 404 that are unmarked by the first labels 402 do not correspond to nuclei of the cells. For example, such pixels could correspond to locations outside of the cells or within cytoplasm of the cells.

Figure 4 shows an additional exemplary functionality related to training the computational model 400. More specifically, Figure 4 shows the results of a more automated process for labeling the first pixels 404 of the fluorescence images 406, taking the form of a binary map 410. The binary map 410 is a compressed form of the fluorescence image 406 shown in Figure 3, as described below. The binary map 410 in Figure 4 serves as an example for many binary maps 410 corresponding to many fluorescence images 406.

As shown, the computing device 200 generates the first labels 402 for the first pixels 404 of the fluorescence images 406 of the samples 408. The first labels 402 indicate whether the first pixels 404 represent a nucleus within the samples 408. That is, the first labels 402 correspond to the first pixels 404 that do represent a nucleus within the sample 408 and the other first pixels 404 do not represent a nucleus within the sample 408. In practice, the first labels 402 will have the same shape and location in both the fluorescence image 406 and the corresponding binary map 410, but Figures 3 and 4 serve as suitable examples.

To generate the first labels 402 of the binary map 410, the computing device 200 may perform a thresholding process on intensities of the first pixels 404 of the fluorescence images 406. Regardless of the color model used, methods exist for converting a pixel value defined by a color model into grayscale representing only pixel intensity. Thus, performing the thresholding process includes classifying the first pixels 404 into nuclear pixels (e.g., indicated by the first labels 402 in the binary map 410) and non-nuclear pixels based on whether the intensities of the first pixels 404 exceed a threshold intensity. Nuclear pixels will generally be brighter than non-nuclear pixels due to the fluorescent nuclear markers that have been used to identify the nuclear area of the cells. (In Figures 3 and 4, nuclear pixels are actually darker than non-nuclear pixels for ease of illustration.) For example, pixel intensity could be defined on a scale from 0 to 1, and the threshold value could be 0.8. Thus, the first pixels 404 having an intensity greater than 0.8 would be considered nuclear pixels and the first pixels 404 having an intensity less than or equal to 0.8 would be considered non-nuclear pixels. The computing device 200 applies the first labels 402 (e.g., only) to locations that correspond with the nuclear pixels. Other intensity scales and threshold intensities are possible.

Additionally, the threshold value could be redefined or adjusted based on manual inspection. For example, a human can review the results of the thresholding process and may determine that using a different threshold intensity for the thresholding process would more accurately classify the first pixels 404 into nuclear pixels and non-nuclear pixels. In one embodiment, the computing device 200 requests, via a user interface, input indicating a second threshold intensity. The request can take the form of a displayed user prompt, for example. Next, the computing device 200 receives the input indicating a (e.g., new) second threshold intensity and reclassifies the first pixels 404 into the nuclear pixels and the non-nuclear pixels based on whether the intensities of the first pixels 404 exceed the second threshold intensity. The user generally decides to end this process when the user determines that the updated threshold intensity has been optimized to accurately classify nuclear and non-nuclear pixels.

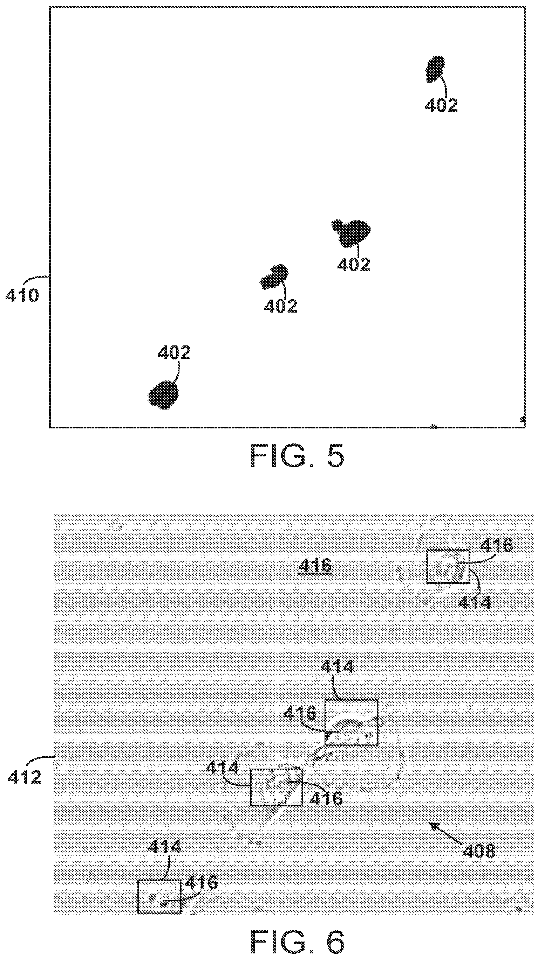

Figure 5 shows an exemplary binary map 410, and Figure 6 shows an exemplary non-fluorescence image 412 of the sample 408. The non-fluorescence image 412 in Figure 6 serves as an example for many non-fluorescence images 412 corresponding to the many samples 408.

In this context, the computing device 200 generates, based on the first labels 402 (e.g., of the binary map 410), second labels 414 for second pixels 416 of the non-fluorescence images 412 of the samples 408. The second labels 414 indicate whether the second pixels 416 represent a nucleus within the samples 408. In practice, the second labels 4 14 will actually have the same shape and location as the first labels 402, but Figure 6 serves as a suitable example. Thus, generating the second labels 414 can include applying locations and shapes of the first labels 402 within the binary map 410 to the non-fluorescence images 412.

Next, the computing device 200 trains the computational model 400 to identify pixels of non-fluorescence images that represent nuclei using the second labels 414 and the non-fluorescence images 412.

That is, the computational model 400 evaluates the second labels 414 and the non-fluorescence images 412 (e.g., the second pixels 416) to identify common attributes of the second pixels 416 labeled as corresponding to a nucleus. Then, the computing device 200 uses those attributes to more accurately classify unlabeled pixels of non-fluorescence images as nuclear or non-nuclear. High pixel intensity makes it more likely that the computational model 400 will label that unlabeled pixel as nuclear. More generally, the computing device 200 will adjust various weighting factors that correspond to algorithms of the computational model 400 based on evaluation of the second labels 414 and the non-fluorescence images 412 so that the computational model 400 is more accurate in classifying unlabeled pixels as nuclear or non-nuclear.

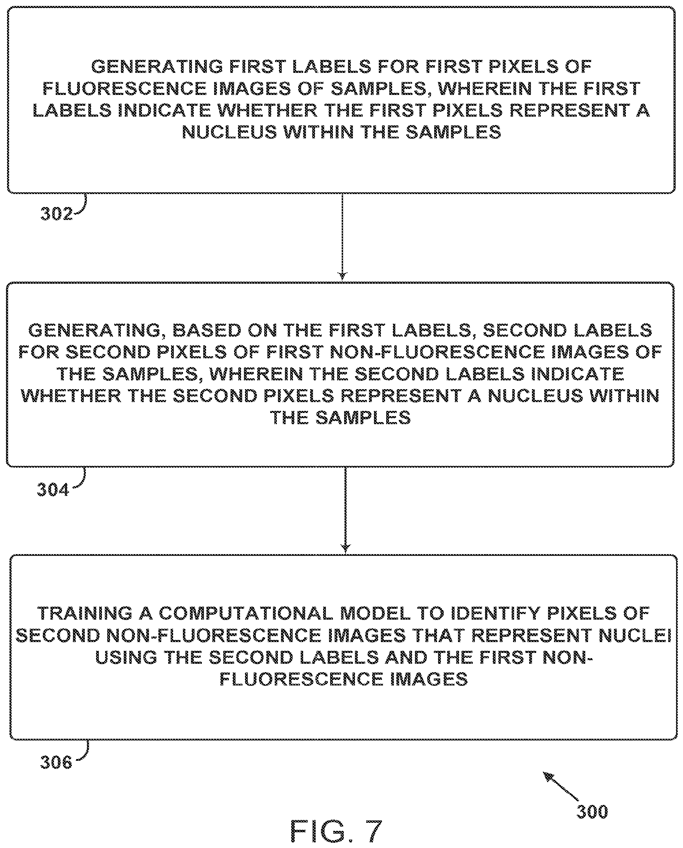

Figure 7 is a block diagram of a method 300 for training the computational model 400. As shown in Figure 7, the method 300 includes one or more operations, functions, or actions as illustrated by blocks 302, 304, and 306. Although the blocks are illustrated in a sequential order, these blocks may also be performed in parallel, and/or in a different order than those described herein. Also, the various blocks may be combined into fewer blocks, divided into additional blocks, and/or removed based upon the desired implementation.

At block 302, the method 300 includes generating the first labels 402 for the first pixels 404 of the fluorescence images 406 of the samples 408, where the first labels 402 indicate whether the first pixels 404 represent a nucleus within the samples 408. Block 302 is described above with reference to Figure 3 and Figure 4.

At block 304, the method 300 includes generating, based on the first labels 402, the second labels 414 for the second pixels 416 of the non-fluorescence images 412 of the samples 408, where the second labels 414 indicate whether the second pixels 416 represent a nucleus within the samples 408. Block 304 is described above with reference to Figure 5 and Figure 6.

At block 306, the method 300 includes training the computational model 400 to identify pixels of non-fluorescence images that represent nuclei using the second labels 414 and the non-fluorescence images 412. Block 306 is described above with reference to Figure 6.

**Using the Trained Computational Model**

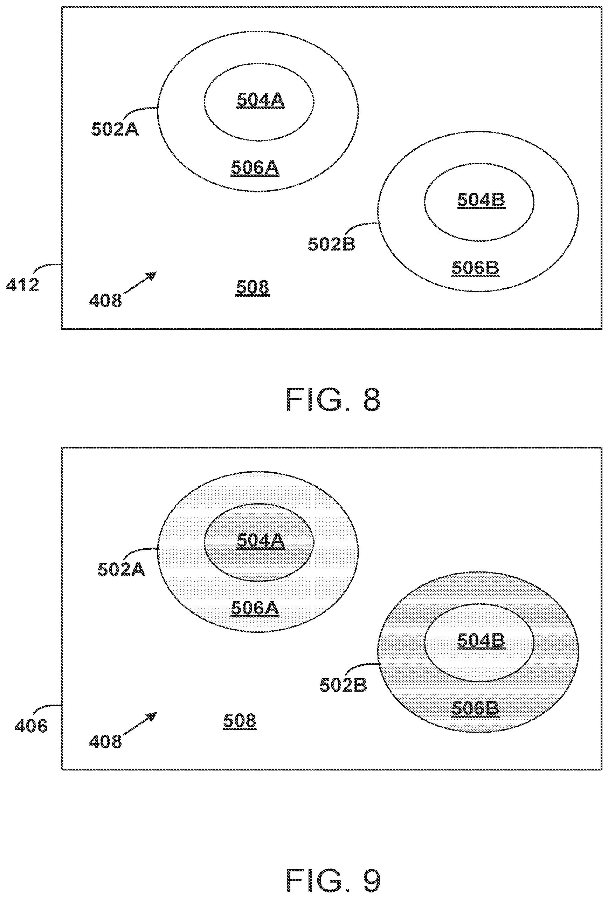

Referring to Figure 8, the optical microscope 105 captures the non-fluorescence image 412 of the sample 408 that includes a live cell 502A and a live cell 502B. Any suitable live nucleated cells may be used. In one embodiment, the one or more cells are adherent cells. In another embodiment, the one or more cells may be mammalian cells. In another embodiment, the one or more mammalian cells are adherent mammalian cells. The one or more cells may all be the same cell type or may include different cell types. This is referred to in the claims below as step (a). The cell 502A includes a nucleus 504A and cytoplasm 506A. The cell 502B includes a nucleus 504B and cytoplasm 506B. The sample 408 also includes a background region 508 that includes any portion of the sample 408 that is not part of the cell 502A or the cell 502B.

The cell 502A (e.g., the nucleus 504A and the cytoplasm 506A) and the cell 502B (e.g., the nucleus 504B and the cytoplasm 506B) of the sample 408 include varying concentrations of protein-based nuclear translocation reporters that can be used to monitor various cellular processes. As used herein, a "fluorescent protein-based nuclear translocation reporter" is a fusion protein comprising a fluorescent protein and a protein that shuttles into and/or out of the nucleus in response to a stimulus of interest. Any suitable fluorescent protein may be used as deemed appropriate for an intended use, including but not limited to green fluorescent protein, red fluorescent protein, yellow fluorescent protein, blue fluorescent protein, orange fluorescent protein, near infrared fluorescent protein, and any derivatives thereof. For example, derivatives of green fluorescent protein (GFP) include, but are not limited to, EGFP, Emerald, Superfolder GFP, Azami Green, mWasabi, TagGFP, TurboGFP, AcGFP, ZsGreen, and T-Sapphire. In one exemplary embodiment, the fluorescent protein-based nuclear translocation reporters (FTRs) may comprise protein kinase translocation reporters, which include any reporters that move into or out of the nucleus in response to protein kinase and phosphatase activity in the cells. In one embodiment, the protein kinase translocation reporters may comprise human FoxO1 protein fused to a fluorescent protein including but not limited to TagGFP2. In this embodiment the reporter may be referred to as "KTR", and the protein kinase being monitored is Akt, as FoxO1 is phosphorylated by active Akt kinase. When Akt is active, KTR phosphorylation makes nuclear export strong, and the KTR is localized to the cytoplasm. When Akt is inactive, KTR dephosphorylation leads to nuclear export becoming weaker than nuclear import, and the KTR is localized to the nuclei. Akt activity can thus be quantified using the ratio of nuclear: cytoplasmic (or nuclear: whole cell) fluorescence.

Non-limiting examples of other FTRs include, but are not limited to, phosphatase translocation reporters (responsive to phosphatase activity in the cells), protease translocation reporters (responsive to protease activity in the cells), and analyte responsive translocation reporters, wherein the analyte may be any analyte including hydrogen ions, potassium ions, calcium ions, etc.. The FTRs are typically indiscernible in the non-fluorescence image 412 because the fluorescence of the FTRs are usually dominated by the other captured light.

The FTRs are expressed by the cells and may be encoded by any vector capable of expressing the FTRs in cells to be used in the methods of the disclosure. Any suitable method to introduce the expression vector into the cells may be used. In one embodiment, the cells may be transiently transfected to introduce the expression vector. In other embodiments, the cells may be stably transfected (such as via viral infection, for example using a lentivirus) to permit stable expression of the FTR in the cells.

The computing device 200 uses the computational model 400 to identify nuclear pixels of the non-fluorescence image 412 that correspond to the nucleus 504A or the nucleus 504B. This is referred to below as step (c). As described above, the computational model 400 has been trained to recognize nuclear pixels within unlabeled non-fluorescence images.

Referring to Figure 9, the fluorescence microscope 107 captures a fluorescence image 406 of the FTRs in the sample 408 (e.g., in the cell 502A and the cell 502B). This is referred to below as step (b). Because fluorescent imaging selectively captures fluorescence emitted from the FTRs within the sample 408, regions of the sample 408 have different brightness levels due to the varying concentrations of the FTRs within the sample 408. As illustrated by varying levels of gray within Figure 9, the nucleus 504A and the cytoplasm 506B have high concentrations of the FTRs, the background region 508 has a near zero level of the FTRs, and the cytoplasm 506A and the nucleus 504B have low levels of the FTRs. In other examples, high FTR intensity can be correlated with lighter shades of gray, however Figure 9 maps low FTR intensity to lighter shades of gray for ease of illustration.

The computing device 200 uses the nuclear pixels (e.g., pixels corresponding to the nucleus 504A and the nucleus 504B) of the non-fluorescence image 412 to identify first pixels of the fluorescence image 406 that correspond to the nucleus 504A or the nucleus 504B and second pixels of the fluorescence image 406 that do not correspond to the nucleus 504A or the nucleus 504B. This is referred to below as step (d). Since the fluorescence image 406 and the non-fluorescence image 412 have the same field of view, a binary map of the nuclear pixels of the non-fluorescence image 412 can be applied to the fluorescence image 406 to identify the first pixels of the fluorescence image 406 that correspond to the nucleus 504A or the nucleus 504B.

Next, the computing device 200 calculates, based on first intensities of the first pixels (e.g., the pixels corresponding to the nucleus 504A and/or the nucleus 504B in the fluorescence image 406) and second intensities of the second pixels (the pixels corresponding to the background region 508, the cytoplasm 506A, and/or the cytoplasm 506B in the fluorescence image 406), a metric representing a first amount of the FTRs located within the nucleus 504A and/or the nucleus 504B and a second amount of the FTRs not located within the nucleus 504A or the nucleus 504B. This is referred to below as step (e).

Generally, the metric will take the form of a ratio, but the metric could also take the form of a difference. Other examples are possible. When the metric is described as representing a first amount of FTRs located within nuclei and a second amount of FTRs not located within nuclei, it can mean that the ratio of the first amount of FTRs located within nuclei and the second amount of FTRs not located within nuclei is derivable from the metric, if not directly expressed by the metric.

The sample 408 can be fully characterized as the union of the background region 508, the cell 502A, and the cell 502B. In other examples, the sample 408 may include many more cells as well. Therefore, the sample 408 in this example can be expressed mathematically as:

*sample* 408 = *background* 508 + *nucleus* 504*A* + *cytoplasm* 506*A* + *nucleus* 504*B* + *cytoplasm* 506*B*

Thus, the metric R can the take the form of:

$$R = G(F(504A, 504B), F(508, 506A, 506B))$$

where F is a function that yields a sum, an average (e.g., a mean), or a median of its arguments and G is a function that yields a ratio or a difference of its arguments. Generally, multiple instances of the function F will take the same form (e.g., sum, average, or mean) in a given example of the metric R.

Thus, in this example, the metric R can be the sum, average, or median of the intensities of the pixels of the nucleus 504A and the nucleus 504B, divided by or subtracted from the sum, average, or median of the intensities of the pixels of the *background* 508, the cytoplasm 506A, and the cytoplasm 506B. Based on these principles, the metric R can take many other forms as well:

$$R = G(F(504A + 504B), F(508 + 506A + 506B + 504A + 504B))$$

$$R = G(F(506A + 506B), F(508 + 506A + 506B + 504A + 504B))$$

In some examples, the computing device 200 segments *background* from cells in the non-fluorescence image 412 of the sample 408 and excludes the second pixels not belonging to cells from the calculating of the second intensities of the second pixels. Thus, the metric R can also take the following forms:

$$R = G(F(504A, 504B), F(506A, 506B))$$

$$R = G(F(504A + 504B), F(506A + 506B + 504A + 504B))$$

$$R = G(F(506A + 506B), F(506A + 506B + 504A + 504B))$$

In some examples, a single cell may be of interest. Accordingly, the computing device 200 may identify (e.g., via a clustering algorithm) the nuclear pixels of the non-fluorescence image 412 that correspond to the nucleus 504A and identify (e.g., via a clustering algorithm), within the fluorescence image 406, the first pixels that correspond to the nucleus 504A and the second pixels that are within the cytoplasm 506A. Thus, in some examples, the metric R may only pertain to a single cell:

$$R = G(F(504A), F(508, 506A))$$

$$R = G(F(504A), F(508 + 506A + 504A))$$

$$R = G(F(506A), F(508 + 506A + 504A))$$

$$R = G(F(504A), F(506A))$$

$$R = G(F(504A), F(506A + 504A))$$

$$R = G(F(506A), F(506A + 504A))$$

As will be understood by those of skill in the art, the methods of the disclosure can be used to assess the effect of a test compound on an activity that the FTR is responsive to. Thus, in one embodiment, the sample 408 is contacted with a test compound, and the computing device 200 performs steps (a)-(e) a plurality of times (e.g., over a period of time) to determine an effect of the test compound on the first amount of the FTRs located within the nucleus 504A and/or the nucleus 504B and the second amount of the FTRs not located within the nucleus 504A and/or the nucleus 504B. Any suitable test compound may be used, including but not limited to, small molecules, proteins, peptides, nucleic acids, lipids, carbohydrates, etc. The effect of the test compound on localization of the FTRs provides a measure of the test compound effect on the activity that the FTR is responsive to.

In some examples, the metric R provides a measure of kinase, phosphatase, or protease activity in the cell 502A and/or the cell 502B.

In some examples, the metric R provides a measure of analyte concentration in the cell 502A and/or the cell 502B.

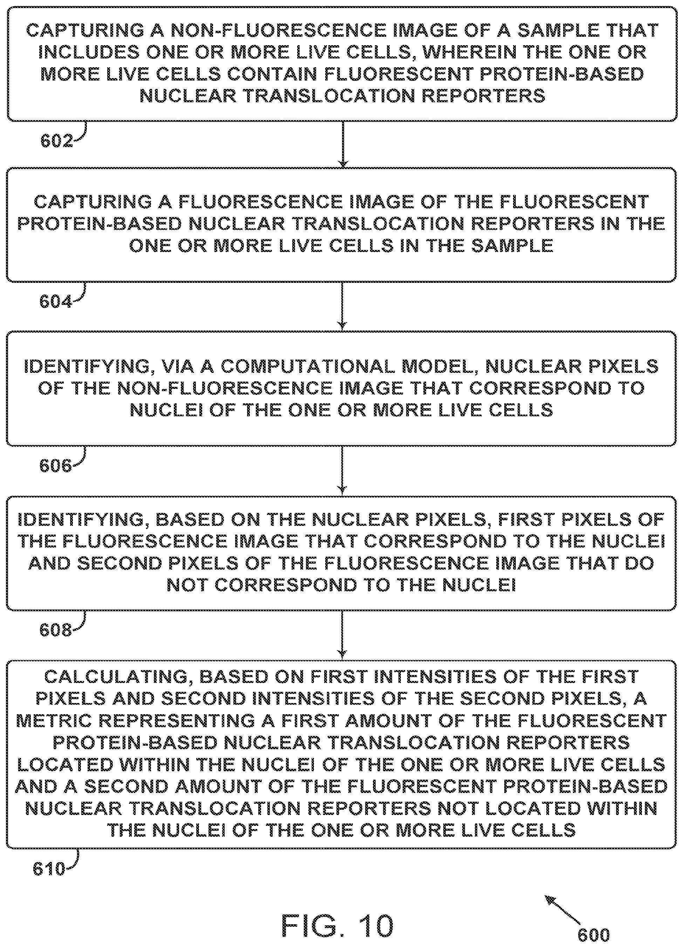

Figure 10 is a block diagram of a method 600 for monitoring the cell 502A and/or the cell 502B. As shown in Figure 10, the method 600 includes one or more operations, functions, or actions as illustrated by blocks 602, 604, 606, 608, and 610. Although the blocks are illustrated in a sequential order, these blocks may also be performed in parallel, and/or in a different order than those described herein. Also, the various blocks may be combined into fewer blocks, divided into additional blocks, and/or removed based upon the desired implementation.

At block 602, the method 600 includes capturing the non-fluorescence image 412 of the sample 408 that includes one or more live cells 502A and 502B.The one or more live cells 502A and 502B may contain fluorescent protein-based nuclear translocation reporters. Block 602 is described above with reference to Figure 8.

At block 604, the method 600 includes capturing the fluorescence image 406 of the fluorescent protein-based nuclear translocation reporters in the one or more live cells 502A and 502B in the sample 408. Block 604 is described above with reference to Figure 9.

At block 606, the method 600 includes identifying, via the computational model 400, nuclear pixels of the non-fluorescence image 412 that correspond to nuclei 504A and 504B of the one or more live cells 502A and 502B. Block 606 is described above with reference to Figure 8.

At block 608, the method 600 includes identifying, based on the nuclear pixels, first pixels of the fluorescence image 406 that correspond to the nuclei 504A and 504B and second pixels of the fluorescence image 406 that do not correspond to the nuclei 504A and 504B. Block 608 is described above with reference to Figure 9.

At block 610, the method 600 includes calculating, based on first intensities of the first pixels and second intensities of the second pixels, a metric R representing a first amount of the fluorescent protein-based nuclear translocation reporters located within the nuclei 504A and 504B of the one or more live cells 502A and 502B and a second amount of the fluorescent protein-based nuclear translocation reporters not located within the nuclei 504A and 504B of the one or more live cells 502A and 502B.Block 610 is described above with reference to Figure 9.

EXPERIMENTAL RESULTS

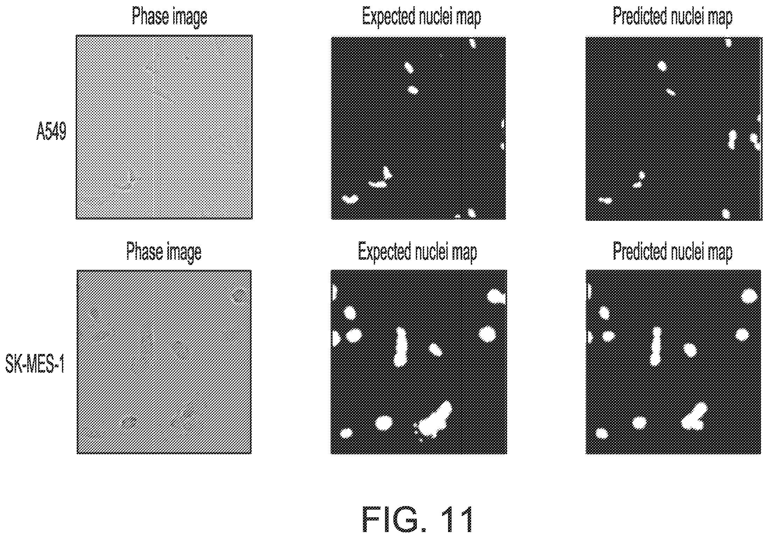

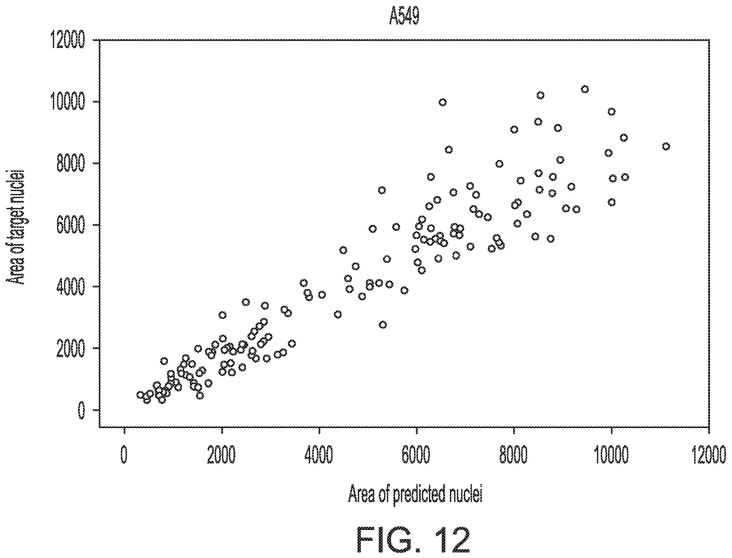

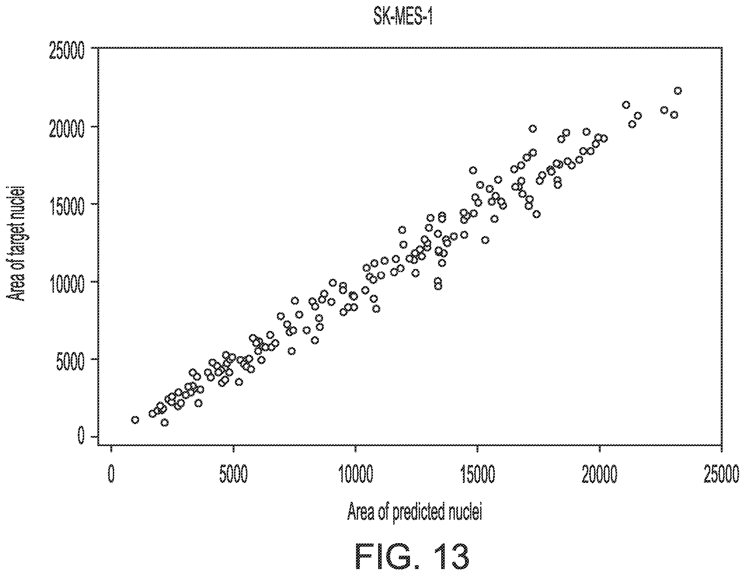

A U-Net-based CNN was trained to detect nuclei of A549 and SK-MES-1 cells. Inputs to the model were phase contrast microscopy images and outputs were binary nuclei maps (see Figure 11). The binary nuclei maps were obtained by pixel-wise thresholding of a fluorescence image of a nuclear marker. Models were trained for 30 epochs for each of the two cell lines to maximize the Dice coefficient between the predicted and expected fluorescence maps, using the Adam optimizer, for a learning rate of 10-4. The nucleus segmentation results were evaluated by comparing the image-wise total areas of marked nuclei of predicted and target images showing strong correlation (R2 = 89% for A549 and 97 % for SK-MES-1, see Figures 12 and 13).

Both nucleus segmentation models can slightly overestimate the nucleus area for two main reasons. Firstly, the sizes of the predicted nuclei are on average slightly larger than the expected nuclei sizes. This issue can be resolved by fine-tuning the threshold selection, model training and post-processing. Secondly, the nuclear markers have < 100 % efficiency, meaning that not all nuclei are marked in the expected nuclei maps. The CNN learn to predict nuclei based on their appearance in the phase contrast image, generally meaning that more nuclei are predicted than present in the target. This issue is especially prominent in the case of the A549-dataset used in this case study and can be mitigated by manual validation of the model predictions by a cell biologist.

The outputs from each model were then used with a fluorescence image to compute how much of the fluorescence were inside the nuclei compared to total fluorescence. To get the predicted nuclei map the output was converted to binary values (pixels belonging to nuclei are equal to 1 and pixels outside of nuclei equal to 0). This map was multiplied pixel-wise with the fluorescence images to get fluorescence inside nuclei. The pixel intensities within predicted nuclei were summed and divided by the sum of the fluorescence intensity of all pixels to get the predicted ratio readout. The same procedure was done with the target nuclei map to get target ratios for comparison.

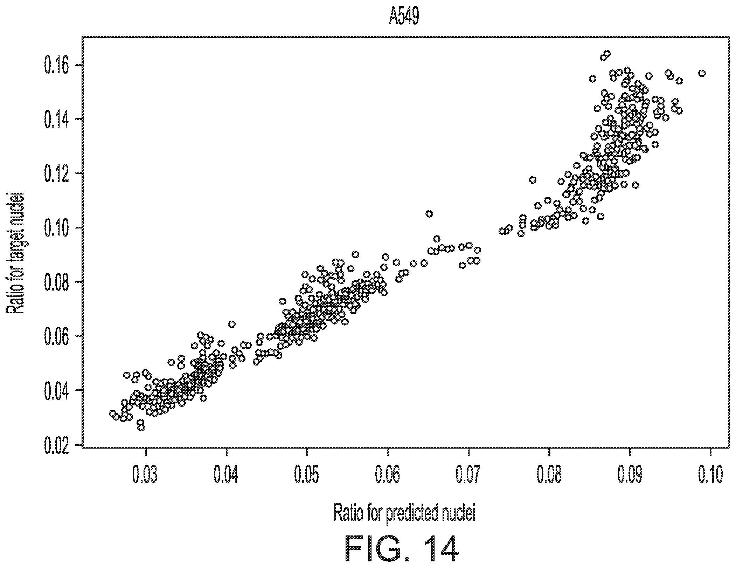

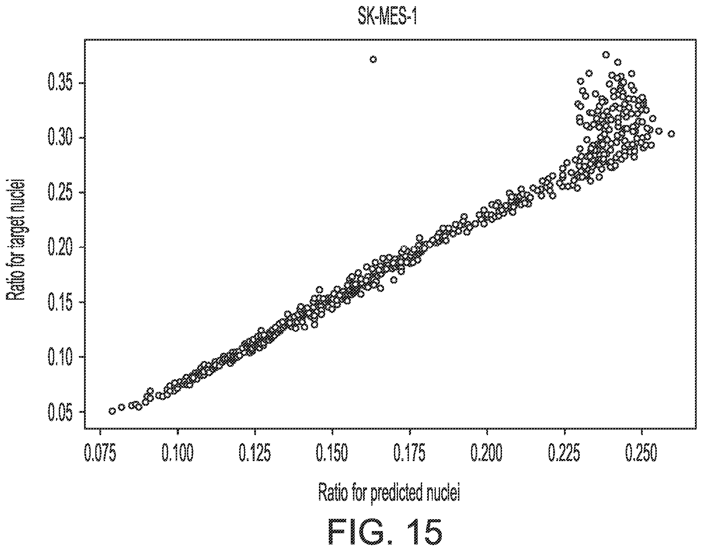

For both cell types, the correlation between ratios acquired using our invention and the compared fluorescence-based ratios was strong (R2 = 97 % for both A549 and SK-MES-1, see Figures 14 and 15) meaning that we can reliably quantify KTR-ratio. Note that the predicted KTR-ratio is underestimated in both cell types, which is a direct consequence of the overestimate nuclei areas shown above and can be solved in two different ways. Firstly, the nuclear sizes can be sorted out as described. Secondly, using a separate calibration set a multiplication factor can be calculated and used to adjust the predicted KTR-ratio.

The KTR ratios were also calculated in a cell-by-cell fashion. In addition to the nucleus segmentation, an instance segmentation model trained to segment individual cells. The instance segmentation model is based on CenterMask and was trained to segment individual cells on a diverse dataset of multiple cell types. Each individual cell segmentation was then used to determine the cell area and ratio to compute the individual cell KTR-ratio like (fluorescence within its nucleus) / (total fluorescence within cell). For this part, cells with a nucleus area below a certain threshold were excluded, this was mostly an issue in the target where some cells had no marking for nucleus or much smaller than expected.

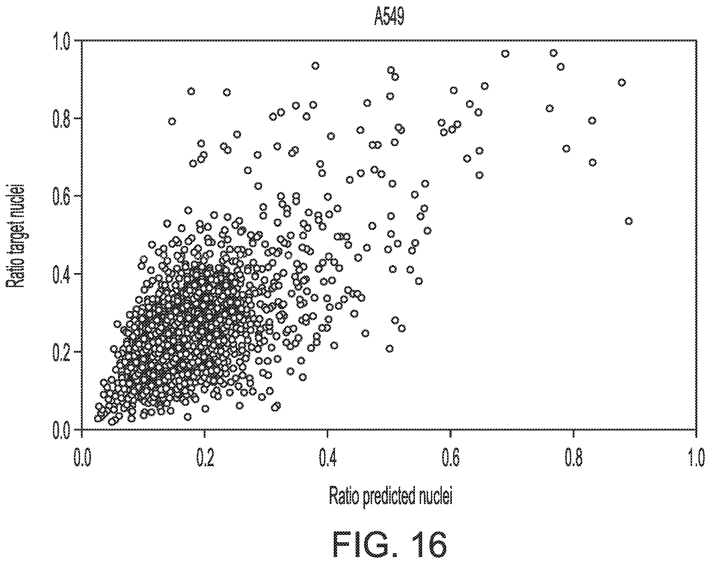

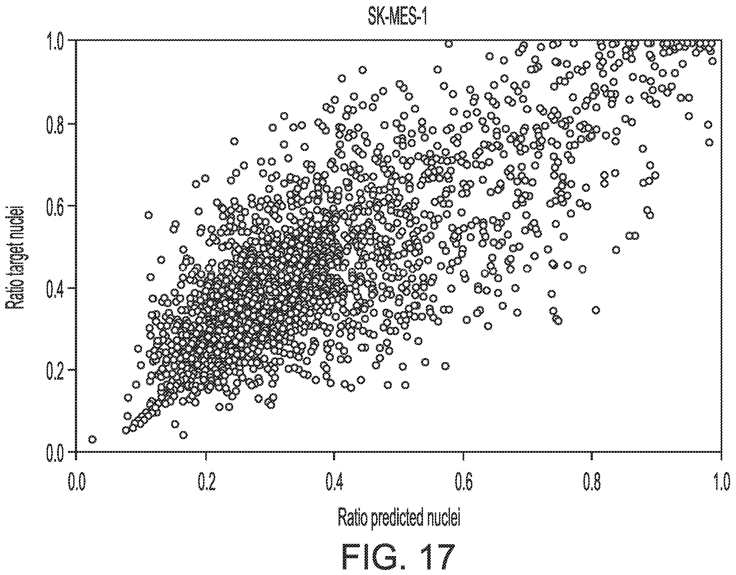

There is a positive correlation for single cell KTR-ratios (R2 = 55 % for A549 and R2 = 78% for SK-MES-1, see Figures 16 and 17) although noisier than whole image KTR-ratios. Even though the noise-level can be improved by fine-tuning both the cell- and nucleus-segmentation models, the results show that we can gain insight into the heterogeneity of KTR-ratios using a single fluorescent KTR marker without needing nucleus or membrane markers to aid segmentation.

The description of different advantageous arrangements has been presented for purposes of illustration and description and is not intended to be exhaustive or limited to the examples in the form disclosed. Many modifications and variations will be apparent to those of ordinary skill in the art. Further, different advantageous examples may describe different advantages as compared to other advantageous examples. The example or examples selected are chosen and described in order to best explain the principles of the examples, the practical application, and to enable others of ordinary skill in the art to understand the disclosure for various examples with various modifications as are suited to the particular use contemplated.

## Claims

1. A method for monitoring one or more live cells, the method comprising:

    (a) capturing a non-fluorescence image of a sample that includes one or more live cells, wherein the one or more live cells contain fluorescent protein-based nuclear translocation reporters;
    (b) capturing a fluorescence image of the fluorescent protein-based nuclear translocation reporters in the one or more live cells in the sample;
    (c) identifying, via a computational model, nuclear pixels of the non-fluorescence image that correspond to nuclei of the one or more live cells;
    (d) identifying, based on the nuclear pixels, first pixels of the fluorescence image that correspond to the nuclei and second pixels of the fluorescence image that do not correspond to the nuclei; and
    (e) calculating, based on first intensities of the first pixels and second intensities of the second pixels, a metric representing a first amount of the fluorescent protein-based nuclear translocation reporters located within the nuclei of the one or more live cells and a second amount of the fluorescent protein-based nuclear translocation reporters not located within the nuclei of the one or more live cells.

2. The method of claim 1, wherein the metric is a ratio of the first amount to the second amount.

3. The method of any one of claims 1-2, wherein the computational model is trained using at least one of non-fluorescence images having pixels labeled as corresponding to a nucleus or not corresponding to a nucleus, and fluorescence images having nuclei tagged with a fluorescent nuclear marker.

4. The method of claim 3, wherein labels for the non-fluorescence images are generated by applying a thresholding algorithm to fluorescence images.

5. The method of any one of claims 1-4, wherein identifying the nuclear pixels comprises generating a binary map that indicates whether each pixel of the non-fluorescence image represents a nucleus.

6. The method of any one of claims 1-5, wherein calculating the metric comprises calculating a sum of the first intensities.

7. The method of claim 6, wherein the sum is a first sum and calculating the metric further comprises:

    calculating a second sum of the second intensities; and
    comparing the first sum to the second sum.

8. The method of any one of claims 1-7, wherein calculating the metric comprises calculating an average of the first intensities.

9. The method of claim 8, wherein the average is a first average and calculating the metric further comprises:

   calculating a second average of the second intensities; and
   comparing the first average to the second average.

10. The method of any one of claims 1-9, wherein the second pixels correspond to cytoplasm of the one or more live cells, and wherein the calculating comprises calculating, based on the first intensities of the first pixels and the second intensities of the second pixels, the metric representing the first amount of the fluorescent protein-based nuclear translocation reporters located within the nuclei and the second amount of the fluorescent protein-based nuclear translocation reporters located within the cytoplasm of the one or more live cells.

11. The method of any one of claims 1-9, wherein the calculating comprises calculating, based on the first intensities of the first pixels and the second intensities of the second pixels, the metric representing the first amount of the fluorescent protein-based nuclear translocation reporters located within the nuclei and the second amount of the fluorescent protein-based nuclear translocation reporters located within the one or more live cells.

12. The method of any one of claims 1-9, wherein the second pixels correspond to cytoplasm of the one or more live cells, and wherein the calculating comprises calculating, based on the first intensities of the first pixels and the second intensities of the second pixels, the metric representing the second amount of the fluorescent protein-based nuclear translocation reporters located within the cytoplasm and a third amount of the fluorescent protein-based nuclear translocation reporters located within the one or more live cells.

13. The method of any one of claims 1-12, wherein the fluorescent protein-based nuclear translocation reporters are selected from the group consisting of protein kinase translocation reporters, phosphatase translocation reporters, protease translocation reporters, and analyte responsive translocation reporters.

14. The method of any one of claims 1-13, further comprising segmenting background from cells in the non-fluorescence image of the sample, and excluding the second pixels not belonging to cells from the calculating of the second intensities of the second pixels.

15. The method of any one of claims 1-14, wherein the method further comprises contacting the sample with a test compound, and carrying out steps (a)-(e) a plurality of times to determine an effect of the test compound on the first amount of the fluorescent protein-based nuclear translocation reporters located within the nuclei of the one or more live cells and the second amount of the fluorescent protein-based nuclear translocation reporters not located within the nuclei of the one or more live cells.

16. The method of any one of claims 1-15, wherein:

   identifying the nuclear pixels comprises identifying the nuclear pixels of the non-fluorescence image that correspond to a single nucleus of a single cell of the one or more live cells,
   identifying the first pixels and the second pixels comprises identifying the first pixels that correspond to the single nucleus and the second pixels that are within a cytoplasm of the single cell, and
   calculating the metric comprises calculating the metric that represents the first amount of the fluorescent protein-based nuclear translocation reporters located within the nucleus and the second amount of the fluorescent protein-based nuclear translocation reporters located within the cytoplasm of the single cell.

17. The method of any one of claims 1-15, wherein:

   identifying the nuclear pixels comprises identifying the nuclear pixels of the non-fluorescence image that correspond to a single nucleus of a single cell of the one or more live cells,
   identifying the first pixels and the second pixels comprises identifying the first pixels that correspond to the nucleus and the second pixels that are within a cytoplasm of the single cell, and
   calculating the metric comprises calculating the metric that represents the first amount of the fluorescent protein-based nuclear translocation reporters located within the single nucleus and a third amount of the fluorescent protein-based nuclear translocation reporters located within the single cell.

18. The method of any one of claims 1-15, wherein:

identifying the nuclear pixels comprises identifying the nuclear pixels of the non-fluorescence image that correspond to a single nucleus of a single cell of the one or more live cells,

identifying the first pixels and the second pixels comprises identifying the first pixels that correspond to the nucleus and the second pixels that are within a cytoplasm of the single cell, and

calculating the metric comprises calculating the metric representing the second amount of the fluorescent protein-based nuclear translocation reporters located within the cytoplasm of the single cell and a third amount of the fluorescent protein-based nuclear translocation reporters located within the single cell.

19. A non-transitory computer readable medium storing instructions that, when executed by a computing device, cause the computing device to perform the method of any one of claims 1-18.

20. A system for monitoring one or more live cells, the system comprising:

an optical microscope;

a fluorescence microscope;

one or more processors; and

a non-transitory computer readable medium storing instructions that, when executed by the one or more processors, cause the system to perform functions comprising:

(a) capturing, via the optical microscope, a non-fluorescence image of a sample that includes one or more live cells, wherein the one or more live cells contain fluorescent protein-based nuclear translocation reporters;

(b) capturing, via the fluorescence microscope, a fluorescence image of the fluorescent protein-based nuclear translocation reporters in the one or more live cells in the sample;

(c) identifying, via a computational model, nuclear pixels of the non-fluorescence image that correspond to nuclei of the one or more live cells;

(d) identifying, based on the nuclear pixels, first pixels of the fluorescence image that correspond to the nuclei and second pixels of the fluorescence image that do not correspond to the nuclei; and

(e) calculating, based on first intensities of the first pixels and second intensities of the second pixels, a metric representing a first amount of the fluorescent protein-based nuclear translocation reporters located within the nuclei of the one or more live cells and a second amount of the fluorescent protein-based nuclear translocation reporters not located within the nuclei of the one or more live cells.

**Patentansprüche**

1. Verfahren zur Überwachung einer oder mehrerer lebender Zellen, wobei das Verfahren umfasst:

(a) Aufnehmen eines Nicht-Fluoreszenzbildes einer Probe, die eine oder mehrere lebende Zellen umfasst, wobei die eine oder die mehreren lebenden Zellen auf fluoreszierenden Proteinen basierende nukleäre Translokationsreporter enthalten;

(b) Aufnehmen eines Fluoreszenzbildes der auf fluoreszierenden Proteinen basierenden nukleären Translokationsreporter in der einen oder den mehreren lebenden Zellen in der Probe;

(c) Identifizieren, mittels eines Rechenmodells, von Kernpixeln des Nicht-Fluoreszenzbildes, die Zellkernen der einen oder der mehreren lebenden Zellen entsprechen;

(d) Identifizieren, auf der Grundlage der Kernpixel, von ersten Pixeln des Fluoreszenzbildes, die den Zellkernen entsprechen, und von zweiten Pixeln des Fluoreszenzbildes, die nicht den Zellkernen entsprechen; und

(e) Berechnen, auf der Grundlage von ersten Intensitäten der ersten Pixel und von zweiten Intensitäten der zweiten Pixel, einer Metrik, die eine erste Menge der auf fluoreszierenden Proteinen basierenden nukleären Translokationsreporter, die sich innerhalb der Zellkerne der einen oder der mehreren lebenden Zellen befinden, und eine zweite Menge der auf fluoreszierenden Proteinen basierenden nukleären Translokationsreporter, die sich nicht innerhalb der Zellkerne der einen oder der mehreren lebenden Zellen befinden, repräsentiert.

2. Verfahren nach Anspruch 1, wobei die Metrik ein Verhältnis der ersten Menge zur zweiten Menge ist.

3. Verfahren nach einem der Ansprüche 1 bis 2, wobei das Rechenmodell unter Verwendung von mindestens einem Nicht-Fluoreszenzbild mit Pixeln, die als einem Zellkern entsprechend oder nicht einem Zellkern entsprechend etikettiert sind, und Fluoreszenzbildern mit Zellkernen, die mit einem fluoreszierenden Kernmarker markiert sind, trainiert ist.

4. Verfahren nach Anspruch 3, wobei die Etiketten für die Nicht-Fluoreszenzbilder durch Anwenden eines Schwellenwertalgorithmus auf Fluoreszenzbilder erzeugt werden.

5. Verfahren nach einem der Ansprüche 1 bis 4, wobei das Identifizieren der Kernpixel das Erzeugen einer Binärkarte umfasst, die angibt, ob jeder Pixel des Nicht-Fluoreszenzbildes einen Zellkern repräsentiert.

6. Verfahren nach einem der Ansprüche 1 bis 5, wobei das Berechnen der Metrik das Berechnen einer Summe der ersten Intensitäten umfasst.

7. Verfahren nach Anspruch 6, wobei die Summe eine erste Summe ist und das Berechnen der Metrik ferner umfasst:

Berechnen einer zweiten Summe der zweiten Intensitäten; und
Vergleichen der ersten Summe mit der zweiten Summe.

8. Verfahren nach einem der Ansprüche 1 bis 7, wobei das Berechnen der Metrik das Berechnen eines Durchschnitts der ersten Intensitäten umfasst.

9. Verfahren nach Anspruch 8, wobei der Durchschnitt ein erster Durchschnitt ist und das Berechnen der Metrik ferner umfasst:

Berechnen eines zweiten Durchschnitts der zweiten Intensitäten; und
Vergleichen des ersten Durchschnitts mit dem zweiten Durchschnitt.

10. Verfahren nach einem der Ansprüche 1 bis 9, wobei die zweiten Pixel dem Zytoplasma der einen oder der mehreren lebenden Zellen entsprechen und wobei das Berechnen das Berechnen, auf der Grundlage der ersten Intensitäten der ersten Pixel und der zweiten Intensitäten der zweiten Pixel, der Metrik, die die erste Menge der auf fluoreszierenden Proteinen basierenden nukleären Translokationsreporter, die sich innerhalb der Zellkerne befinden, und die zweite Menge der auf fluoreszierenden Proteinen basierenden nukleären Translokationsreporter, die sich innerhalb des Zytoplasmas der einen oder der mehreren lebenden Zellen befinden, repräsentiert, umfasst.

11. Verfahren nach einem der Ansprüche 1 bis 9, wobei das Berechnen das Berechnen, auf der Grundlage der ersten Intensitäten der ersten Pixel und der zweiten Intensitäten der zweiten Pixel, der Metrik, die die erste Menge der auf fluoreszierenden Proteinen basierenden nukleären Translokationsreporter, die sich innerhalb der Zellkerne befinden, und die zweite Menge der auf fluoreszierenden Proteinen basierenden nukleären Translokationsreporter, die sich innerhalb der einen oder der mehreren lebenden Zellen befinden, repräsentiert, umfasst.

12. Verfahren nach einem der Ansprüche 1 bis 9, wobei die zweiten Pixel dem Zytoplasma der einen oder der mehreren lebenden Zellen entsprechen und wobei das Berechnen das Berechnen, auf der Grundlage der ersten Intensitäten der ersten Pixel und der zweiten Intensitäten der zweiten Pixel, der Metrik, die die zweite Menge der auf fluoreszierenden Proteinen basierenden nukleären Translokationsreporter, die sich innerhalb des Zytoplasmas befinden, und eine dritte Menge der auf fluoreszierenden Proteinen basierenden nukleären Translokationsreporter, die sich innerhalb der einen oder der mehreren lebenden Zellen befinden, repräsentiert, umfasst.

13. Verfahren nach einem der Ansprüche 1 bis 12, wobei die auf fluoreszierenden Proteinen basierenden nukleären Translokationsreporter ausgewählt sind aus der Gruppe bestehend aus Proteinkinase- Translokationsreportern, Phosphatase-Translokationsreportern, Protease-Translokationsreportern und analytresponsiven Translokationsreportern.

14. Verfahren nach einem der Ansprüche 1 bis 13, ferner umfassend das Segmentieren des Hintergrunds von den Zellen in dem Nicht-Fluoreszenzbild der Probe und das Ausschließen der nicht zu Zellen gehörenden zweiten Pixel aus dem Berechnen der zweiten Intensitäten der zweiten Pixel.

15. Verfahren nach einem der Ansprüche 1 bis 14, wobei das Verfahren ferner das Inkontaktbringen der Probe mit einer Testverbindung und das Durchführen der Schritte (a) bis (e) eine Vielzahl von Malen umfasst, um eine Wirkung der Testverbindung auf die erste Menge der auf fluoreszierenden Proteinen basierenden nukleären Translokationsreporter, die sich innerhalb der Zellkerne der einen oder der mehreren lebenden Zellen befinden, und die zweite Menge der auf fluoreszierenden Proteinen basierenden nukleären Translokationsreporter, die sich nicht innerhalb der Zellkerne der einen oder der mehreren lebenden Zellen befinden, zu bestimmen.

16. Verfahren nach einem der Ansprüche 1 bis 15, wobei:

> das Identifizieren der Kernpixel das Identifizieren der Kernpixel des Nicht-Fluoreszenzbildes, die einem einzelnen Zellkern einer einzelnen Zelle der einen oder der mehreren lebenden Zellen entsprechen, umfasst,
> das Identifizieren der ersten Pixel und der zweiten Pixel das Identifizieren der ersten Pixel, die dem einzelnen Zellkern entsprechen, und der zweiten Pixel, die sich innerhalb eines Zytoplasmas der einzelnen Zelle befinden, umfasst, und
> das Berechnen der Metrik das Berechnen der Metrik, die die erste Menge der auf fluoreszierenden Proteinen basierenden nukleären Translokationsreporter, die sich innerhalb des Zellkerns befinden, und die zweite Menge der auf fluoreszierenden Proteinen basierenden nukleären Translokationsreporter, die sich innerhalb des Zytoplasmas der einzelnen Zelle befinden, repräsentiert, umfasst.

17. Verfahren nach einem der Ansprüche 1 bis 15, wobei:

> das Identifizieren der Kernpixel das Identifizieren der Kernpixel des Nicht-Fluoreszenzbildes, die einem einzelnen Zellkern einer einzelnen Zelle der einen oder der mehreren lebenden Zellen entsprechen, umfasst,
> das Identifizieren der ersten Pixel und der zweiten Pixel das Identifizieren der ersten Pixel, die dem Zellkern entsprechen, und der zweiten Pixel, die sich innerhalb eines Zytoplasmas der einzelnen Zelle befinden, umfasst, und
> das Berechnen der Metrik das Berechnen der Metrik, die die erste Menge der auf fluoreszierenden Proteinen basierenden nukleären Translokationsreporter, die sich innerhalb des einzelnen Zellkerns befinden, und eine dritte Menge der auf fluoreszierenden Proteinen basierenden nukleären Translokationsreporter, die sich innerhalb der einzelnen Zelle befinden, repräsentiert, umfasst.

18. Verfahren nach einem der Ansprüche 1 bis 15, wobei:

> das Identifizieren der Kernpixel das Identifizieren der Kernpixel des Nicht-Fluoreszenzbildes, die einem einzelnen Zellkern einer einzelnen Zelle der einen oder der mehreren lebenden Zellen entsprechen, umfasst,
> das Identifizieren der ersten Pixel und der zweiten Pixel das Identifizieren der ersten Pixel, die dem Zellkern entsprechen, und der zweiten Pixel, die sich innerhalb eines Zytoplasmas der einzelnen Zelle befinden, umfasst, und
> das Berechnen der Metrik das Berechnen der Metrik, die die zweite Menge der auf fluoreszierenden Proteinen basierenden nukleären Translokationsreporter, die sich innerhalb des Zytoplasmas der einzelnen Zelle befinden, und eine dritte Menge der auf fluoreszierenden Proteinen basierenden nukleären Translokationsreporter, die sich innerhalb der einzelnen Zelle befinden, repräsentiert, umfasst.

19. Nichttransitorisches computerlesbares Medium, das Anweisungen speichert, die, wenn sie von einer Rechenvorrichtung ausgeführt werden, die Rechenvorrichtung veranlassen, das Verfahren nach einem der Ansprüche 1 bis 18 auszuführen.

20. System zur Überwachung einer oder mehrerer lebender Zellen, wobei das System umfasst:

> ein optisches Mikroskop;
> ein Fluoreszenzmikroskop;
> einen oder mehrere Prozessoren; und
> ein nichttransitorisches computerlesbares Medium, das Anweisungen speichert, die, wenn sie von dem einen oder den mehreren Prozessoren ausgeführt werden, das System veranlassen, Funktionen auszuführen, umfassend:
>
>> (a) Aufnehmen, mittels des optischen Mikroskops, eines Nicht-Fluoreszenzbildes einer Probe, die eine oder mehrere lebende Zellen umfasst, wobei die eine oder die mehreren lebenden Zellen auf fluoreszierenden Proteinen basierende nukleäre Translokationsreporter enthalten;
>> (b) Aufnehmen, mittels des Fluoreszenzmikroskops, eines Fluoreszenzbildes der auf fluoreszierenden Proteinen basierenden nukleären Translokationsreporter in der einen oder den mehreren lebenden Zellen in der Probe;
>> (c) Identifizieren, mittels eines Rechenmodells, von Kernpixeln des Nicht-Fluoreszenzbildes, die Zellkernen der einen oder der mehreren lebenden Zellen entsprechen;
>> (d) Identifizieren, auf der Grundlage der Kernpixel, von ersten Pixeln des Fluoreszenzbildes, die den Zellkernen entsprechen, und von zweiten Pixeln des Fluoreszenzbildes, die nicht den Zellkernen entsprechen; und

(e) Berechnen, auf der Grundlage von ersten Intensitäten der ersten Pixel und von zweiten Intensitäten der zweiten Pixel, einer Metrik, die eine erste Menge der auf fluoreszierenden Proteinen basierenden nukleären Translokationsreporter, die sich innerhalb der Zellkerne der einen oder der mehreren lebenden Zellen befinden, und eine zweite Menge der auf fluoreszierenden Proteinen basierenden nukleären Translokationsreporter, die sich nicht innerhalb der Zellkerne der einen oder der mehreren lebenden Zellen befinden, repräsentiert.

**Revendications**

1. Procédé de surveillance d'une ou de plusieurs cellules vivantes, le procédé comprenant :

   (a) la capture d'une image de non-fluorescence d'un échantillon qui comprend une ou plusieurs cellules vivantes, dans lequel la une ou les plusieurs cellules vivantes contiennent des rapporteurs de translocation nucléaire à base de protéines fluorescentes ;
   (b) la capture d'une image de fluorescence des rapporteurs de translocation nucléaire à base de protéines fluorescentes dans la une ou les plusieurs cellules vivantes dans l'échantillon ;
   (c) l'identification, par l'intermédiaire d'un modèle computationnel, de pixels nucléaires de l'image de non-fluorescence qui correspondent à des noyaux de la une ou des plusieurs cellules vivantes ;
   (d) l'identification, sur la base des pixels nucléaires, de premiers pixels de l'image de fluorescence qui correspondent aux noyaux et de seconds pixels de l'image de fluorescence qui ne correspondent pas aux noyaux ; et
   (e) le calcul, sur la base de premières intensités des premiers pixels et de secondes intensités des seconds pixels, d'une métrique représentant une première quantité des rapporteurs de translocation nucléaire à base de protéines fluorescentes situés dans les noyaux de la une ou des plusieurs cellules vivantes et une seconde quantité des rapporteurs de translocation nucléaire à base de protéines fluorescentes non situés dans les noyaux de la une ou des plusieurs cellules vivantes.

2. Procédé selon la revendication 1, dans lequel la métrique est un rapport de la première quantité à la seconde quantité.

3. Procédé selon l'une quelconque des revendications 1 à 2, dans lequel le modèle computationnel est entraîné en utilisant au moins une image de non-fluorescence ayant des pixels étiquetés comme correspondant à un noyau ou ne correspondant pas à un noyau, et des images de fluorescence ayant des noyaux marqués avec un marqueur nucléaire fluorescent.

4. Procédé selon la revendication 3, dans lequel les étiquettes pour les images de non-fluorescence sont générées par l'application d'un algorithme de seuillage à des images de fluorescence.

5. Procédé selon l'une quelconque des revendications 1 à 4, dans lequel l'identification des pixels nucléaires comprend la génération d'une carte binaire qui indique si chaque pixel de l'image de non-fluorescence représente un noyau.

6. Procédé selon l'une quelconque des revendications 1 à 5, dans lequel le calcul de la métrique comprend le calcul d'une somme des premières intensités.

7. Procédé selon la revendication 6, dans lequel la somme est une première somme et le calcul de la métrique comprend en outre :

   le calcul d'une seconde somme des secondes intensités ; et
   la comparaison de la première somme à la seconde somme.

8. Procédé selon l'une quelconque des revendications 1 à 7, dans lequel le calcul de la métrique comprend le calcul d'une moyenne des premières intensités.

9. Procédé selon la revendication 8, dans lequel la moyenne est une première moyenne et le calcul de la métrique comprend en outre :

le calcul d'une seconde moyenne des secondes intensités ; et

la comparaison de la première moyenne à la seconde moyenne.

10. Procédé selon l'une quelconque des revendications 1 à 9, dans lequel les seconds pixels correspondent au cytoplasme de la une ou des plusieurs cellules vivantes, et dans lequel le calcul comprend le calcul, sur la base des premières intensités des premiers pixels et des secondes intensités des seconds pixels, de la métrique représentant la première quantité des rapporteurs de translocation nucléaire à base de protéines fluorescentes situés dans les noyaux et la seconde quantité des rapporteurs de translocation nucléaire à base de protéines fluorescentes situés dans le cytoplasme de la une ou des plusieurs cellules vivantes.

11. Procédé selon l'une quelconque des revendications 1 à 9, dans lequel le calcul comprend le calcul, sur la base des premières intensités des premiers pixels et des secondes intensités des seconds pixels, de la métrique représentant la première quantité des rapporteurs de translocation nucléaire à base de protéines fluorescentes situés dans les noyaux et la seconde quantité des rapporteurs de translocation nucléaire à base de protéines fluorescentes situés dans la une ou les plusieurs cellules vivantes.

12. Procédé selon l'une quelconque des revendications 1 à 9, dans lequel les seconds pixels correspondent au cytoplasme de la une ou des plusieurs cellules vivantes, et dans lequel le calcul comprend le calcul, sur la base des premières intensités des premiers pixels et des secondes intensités des seconds pixels, de la métrique représentant la seconde quantité des rapporteurs de translocation nucléaire à base de protéines fluorescentes situés dans le cytoplasme et une troisième quantité des rapporteurs de translocation nucléaire à base de protéines fluorescentes situés dans la une ou les plusieurs cellules vivantes.

13. Procédé selon l'une quelconque des revendications 1 à 12, dans lequel les rapporteurs de translocation nucléaire à base de protéines fluorescentes sont choisis dans le groupe constitué par des rapporteurs de translocation de protéine kinase, des rapporteurs de translocation de phosphatase, des rapporteurs de translocation de protéase et des rapporteurs de translocation sensibles à un analyte.

14. Procédé selon l'une quelconque des revendications 1 à 13, comprenant en outre la segmentation de l'arrière-plan par rapport aux cellules dans l'image de non-fluorescence de l'échantillon, et l'exclusion des seconds pixels n'appartenant pas à des cellules du calcul des secondes intensités des seconds pixels.

15. Procédé selon l'une quelconque des revendications 1 à 14, dans lequel le procédé comprend en outre la mise en contact de l'échantillon avec un composé d'essai, et la réalisation des étapes (a) à (e) une pluralité de fois pour déterminer un effet du composé d'essai sur la première quantité des rapporteurs de translocation nucléaire à base de protéines fluorescentes situés dans les noyaux de la une ou des plusieurs cellules vivantes et la seconde quantité des rapporteurs de translocation nucléaire à base de protéines fluorescentes non situés dans les noyaux de la une ou des plusieurs cellules vivantes.

16. Procédé selon l'une quelconque des revendications 1 à 15, dans lequel :

l'identification des pixels nucléaires comprend l'identification des pixels nucléaires de l'image de non-fluorescence qui correspondent à un noyau unique d'une cellule unique de la une ou des plusieurs cellules vivantes, l'identification des premiers pixels et des seconds pixels comprend l'identification des premiers pixels qui correspondent au noyau unique et des seconds pixels qui sont dans un cytoplasme de la cellule unique, et le calcul de la métrique comprend le calcul de la métrique qui représente la première quantité des rapporteurs de translocation nucléaire à base de protéines fluorescentes situés dans le noyau et la seconde quantité des rapporteurs de translocation nucléaire à base de protéines fluorescentes situés dans le cytoplasme de la cellule unique.

17. Procédé selon l'une quelconque des revendications 1 à 15, dans lequel :

l'identification des pixels nucléaires comprend l'identification des pixels nucléaires de l'image de non-fluorescence qui correspondent à un noyau unique d'une cellule unique de la une ou des plusieurs cellules vivantes, l'identification des premiers pixels et des seconds pixels comprend l'identification des premiers pixels qui correspondent au noyau et des seconds pixels qui sont dans un cytoplasme de la cellule unique, et le calcul de la métrique comprend le calcul de la métrique qui représente la première quantité des rapporteurs de translocation nucléaire à base de protéines fluorescentes situés dans le noyau unique et une troisième quantité des rapporteurs de translocation nucléaire à base de protéines fluorescentes situés dans la cellule unique.

**18.** Procédé selon l'une quelconque des revendications 1 à 15, dans lequel :

l'identification des pixels nucléaires comprend l'identification des pixels nucléaires de l'image de non-fluorescence qui correspondent à un noyau unique d'une cellule unique de la une ou des plusieurs cellules vivantes, l'identification des premiers pixels et des seconds pixels comprend l'identification des premiers pixels qui correspondent au noyau et des seconds pixels qui sont dans un cytoplasme de la cellule unique, et le calcul de la métrique comprend le calcul de la métrique représentant la seconde quantité des rapporteurs de translocation nucléaire à base de protéines fluorescentes situés dans le cytoplasme de la cellule unique et une troisième quantité des rapporteurs de translocation nucléaire à base de protéines fluorescentes situés dans la cellule unique.

**19.** Support lisible par ordinateur non transitoire stockant des instructions qui, lorsqu'elles sont exécutées par un dispositif informatique, amènent le dispositif informatique à exécuter le procédé selon l'une quelconque des revendications 1 à 18.

**20.** Système de surveillance d'une ou de plusieurs cellules vivantes, le système comprenant :

un microscope optique ;
un microscope à fluorescence ;
un ou plusieurs processeurs ; et
un support lisible par ordinateur non transitoire stockant des instructions qui, lorsqu'elles sont exécutées par le un ou les plusieurs processeurs, amènent le système à exécuter des fonctions comprenant :

(a) la capture, par l'intermédiaire du microscope optique, d'une image de non-fluorescence d'un échantillon qui comprend une ou plusieurs cellules vivantes, dans lequel la une ou les plusieurs cellules vivantes contiennent des rapporteurs de translocation nucléaire à base de protéines fluorescentes ;
(b) la capture, par l'intermédiaire du microscope à fluorescence, d'une image de fluorescence des rapporteurs de translocation nucléaire à base de protéines fluorescentes dans la une ou les plusieurs cellules vivantes dans l'échantillon ;
(c) l'identification, par l'intermédiaire d'un modèle computationnel, de pixels nucléaires de l'image de non-fluorescence qui correspondent à des noyaux de la une ou des plusieurs cellules vivantes ;
(d) l'identification, sur la base des pixels nucléaires, de premiers pixels de l'image de fluorescence qui correspondent aux noyaux et de seconds pixels de l'image de fluorescence qui ne correspondent pas aux noyaux ; et
(e) le calcul, sur la base de premières intensités des premiers pixels et de secondes intensités des seconds pixels, d'une métrique représentant une première quantité des rapporteurs de translocation nucléaire à base de protéines fluorescentes situés dans les noyaux de la une ou des plusieurs cellules vivantes et une seconde quantité des rapporteurs de translocation nucléaire à base de protéines fluorescentes non situés dans les noyaux de la une ou des plusieurs cellules vivantes.

FIG. 1

FIG. 2

FIG. 3

FIG. 4

FIG. 5

FIG. 6

GENERATING FIRST LABELS FOR FIRST PIXELS OF FLUORESCENCE IMAGES OF SAMPLES, WHEREIN THE FIRST LABELS INDICATE WHETHER THE FIRST PIXELS REPRESENT A NUCLEUS WITHIN THE SAMPLES

302

GENERATING, BASED ON THE FIRST LABELS, SECOND LABELS FOR SECOND PIXELS OF FIRST NON-FLUORESCENCE IMAGES OF THE SAMPLES, WHEREIN THE SECOND LABELS INDICATE WHETHER THE SECOND PIXELS REPRESENT A NUCLEUS WITHIN THE SAMPLES

304

TRAINING A COMPUTATIONAL MODEL TO IDENTIFY PIXELS OF SECOND NON-FLUORESCENCE IMAGES THAT REPRESENT NUCLEI USING THE SECOND LABELS AND THE FIRST NON-FLUORESCENCE IMAGES

306

300

FIG. 7

FIG. 8

FIG. 9

CAPTURING A NON-FLUORESCENCE IMAGE OF A SAMPLE THAT INCLUDES ONE OR MORE LIVE CELLS, WHEREIN THE ONE OR MORE LIVE CELLS CONTAIN FLUORESCENT PROTEIN-BASED NUCLEAR TRANSLOCATION REPORTERS

602

CAPTURING A FLUORESCENCE IMAGE OF THE FLUORESCENT PROTEIN-BASED NUCLEAR TRANSLOCATION REPORTERS IN THE ONE OR MORE LIVE CELLS IN THE SAMPLE

604

IDENTIFYING, VIA A COMPUTATIONAL MODEL, NUCLEAR PIXELS OF THE NON-FLUORESCENCE IMAGE THAT CORRESPOND TO NUCLEI OF THE ONE OR MORE LIVE CELLS

606

IDENTIFYING, BASED ON THE NUCLEAR PIXELS, FIRST PIXELS OF THE FLUORESCENCE IMAGE THAT CORRESPOND TO THE NUCLEI AND SECOND PIXELS OF THE FLUORESCENCE IMAGE THAT DO NOT CORRESPOND TO THE NUCLEI

608

CALCULATING, BASED ON FIRST INTENSITIES OF THE FIRST PIXELS AND SECOND INTENSITIES OF THE SECOND PIXELS, A METRIC REPRESENTING A FIRST AMOUNT OF THE FLUORESCENT PROTEIN-BASED NUCLEAR TRANSLOCATION REPORTERS LOCATED WITHIN THE NUCLEI OF THE ONE OR MORE LIVE CELLS AND A SECOND AMOUNT OF THE FLUORESCENT PROTEIN-BASED NUCLEAR TRANSLOCATION REPORTERS NOT LOCATED WITHIN THE NUCLEI OF THE ONE OR MORE LIVE CELLS

610

FIG. 10

600

FIG. 11

FIG. 12

EP 4 522 993 B1

SK-MES-1

FIG. 13

FIG. 14

FIG. 15

EP 4 522 993 B1

FIG. 16

FIG. 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 74031322

**Non-patent literature cited in the description**

- **DAVID A. VAN VALEN et al.** Deep Learning Automates the Quantitative Analysis of Individual Cells in Live-Cell Imaging Experiments. *PLOS COMPUTATIONAL BIOLOGY*, 04 November 2016, vol. 12